# EUROPEAN PATENT SPECIFICATION

(11) **EP 2 950 703 B1**
(45) Date of publication and mention of the grant of the patent: **21.03.2018**
(21) Application number: 14746599.1
(22) Date of filing: 31.01.2014
(51) Int. Cl.: A61B 1/018, A61M 5/46

(54) **ENDOSCOPIC INJECTION NEEDLE DEVICE**
ENDOSKOPISCHE INJEKTIONSNADELVORRICHTUNG
DISPOSITIF D'AIGUILLE D'INJECTION ENDOSCOPIQUE

(30) Priority: 31.01.2013 US 201361759347 P
(43) Date of publication of application: 09.12.2015
(73) Proprietor: EndoChoice, Inc., Alpharetta, GA 30009 (US)
(72) Inventor: JACOBS, Charlie, Loganville, GA 30052 (US); TANG, Xiaowei, Nanjing, Jiangsu Province (CN); SHEN, Ning, Nanjing, Jiangsu Province (CN); LENG, Derong, Nanjing, Jiangsu Province (CN)
(74) Representative: Vossius & Partner Patentanwälte Rechtsanwälte mbB
(86) International application number: PCT/US2014/014230
(87) International publication number: WO 2014/121112

(56) References cited:
- EP-A2- 0 868 923
- WO-A1-98/30259
- CN-A- 102 551 846
- CN-A- 102 551 846
- US-A1- 2012 209 063
- US-A1- 2012 259 203
- US-B2- 6 666 847
- US-B2- 7 798 995

## Description

### FIELD

The present specification relates generally to surgical instruments. More particularly, the present specification relates to an endoscopic injection needle device including a spring at its operational end used to stabilize and deploy the needle.

### BACKGROUND

Endoscopic injection needle devices are used to treat a variety of conditions most commonly affecting a portion of the digestive tract. For example, these devices are used to treat esophageal varices through injection sclerotherapy. The needle is inserted into the varices and a chemical agent is injected. The chemical agent scleroses, or hardens, the varices, resulting in tissue necrosis. The necrotic tissue is then reabsorbed by the body. Endoscopic injection needle devices are also used to induce hemostasis in a bleeding gastric ulcer or post sphincterotomy of a blocked bile duct. A chemical agent is injected into the tissue to stop the bleeding. These devices are also used to inject dye into target tissues to mark the tissue. This tattooing is beneficial in the surveillance of target tissues by making it easier for a surgeon to relocate the tissue for observation. These needle devices are also often used to inject saline into the submucosa under a polyp to lift the polyp up from the surrounding tissue, making resection of the polyp far easier.

Typical endoscopic injection needle devices include a flexible, elongate tubular sheath having a lumen attached to the distal end of a handle. A wire or a flexible inner tube, having its own lumen, is disposed within the sheath lumen and extends from the distal end of the handle to the distal end of the sheath. The injection needle is positioned at the distal end of the sheath and is movable from a first configuration within the sheath to a second configuration extending from the sheath. An actuator on the handle is manipulated to advance the inner tube or wire resulting in extension of the needle from the distal end of the sheath. The distal end of the sheath also typically includes a small housing for securing the needle during device placement. The handle includes an injection port that is in fluid communication with the lumen of the sheath in a device having a wire, or with the lumen of the inner tube if present, which is in turn in fluid communication with the needle.

During a procedure using an endoscopic injection needle device, an endoscope is first inserted into a patient with its distal end proximate a target tissue. The needle device is then inserted into a working channel of the endoscope such that the distal end of the needle device extends beyond the distal end of the working channel of the endoscope. The device is maneuvered to have the distal end of the sheath proximate the target tissue. The needle is extended from the sheath by manipulation of the actuator on the handle. The target tissue is then pierced with the needle. Using a syringe attached to the injection port of the handle, a fluid is injected through the device and into the target tissue.

United States Patent Number 6,666,847, assigned to United States Endoscopy Group, Inc., describes a "A surgical device for injecting a chemical agent within a subject for use in endoscopic injection therapies, the device comprising: a. a support body; b. a motion transmitting unit comprising a first end portion proximal to said support body and a second end portion remote from said support body, wherein said motion transmitting unit is movable relative to said support body; c. an agent delivery system comprising: i. a needle remote from said support body having a hollow elongated body, a first end for extending into a subject, and a second end fixed to said motion transmitting unit second end portion; and ii. structure defining a conduit between said support body and said needle; and d. a guide housing for guiding said needle, said housing comprising a flexible elongated body, an end portion proximal to said needle, an internal elongated passage and friction reducing material lining said passage, wherein a part of said motion transmitting unit adjacent said needle is slideably housed within said guide housing; e. wherein said guide housing is constructed from a wire coated with a friction reducing material, said coated wire being helically wound to form said internal elongated passage."

While current endoscopic injection needle devices are effective, they are not without their drawbacks. For example, current needle devices often encounter difficulties with consistent needle deployment. Users often experience sluggish needle deployment and sometimes the needle can inadvertently advance through the sheath wall. In addition, with current devices, needle deployment can become more difficult in tortuous anatomy. Therefore, what is needed is an endoscopic injection needle device with a reinforced distal end and a mechanism that will facilitate needle deployment, ensuring smooth and consistent needle advancement and retraction. CN102551846 discloses a similar injection needle device as defined in the preamble of claim 1.

### SUMMARY

The present specification discloses an injection needle device for use with an endoscope, said device comprising: a handle having a cylindrical body, an exterior surface, a proximal end, and a distal end, said handle further comprising a first locking member on said exterior surface and a compressible spring within said cylindrical body; an actuator having a proximal end and a distal end, wherein said actuator is coupled at said proximal end to the distal end of said cylindrical body of said handle, said actuator further comprising a second locking member having a plurality of locking components, each of said locking components being separated by an interval; a spring coil; a drive shaft having a first lumen, a proximal end, and a distal end, wherein said proximal end of said drive shaft attaches to said distal end of said actuator and wherein said distal end of said shaft extends within said spring coil; and a needle attached to said distal end of said drive shaft, wherein said actuator is configured such that, upon advancement, said first locking member traverses said plurality of locking components of said second locking member and intervals therebetween, engaging one of the plurality of locking components of said second locking member to lock said actuator in place and said proximal end of said actuator telescopes into said body of said handle and compresses said compressible spring causing said needle to move from a first position to a second position, separated by a distance, wherein the distance is a predefined distance that is determinable based upon a number of said intervals traversed by the first locking member.

In one embodiment, the injection needle device further comprises a plurality of ridges on at least one side of said exterior surface of said handle to form a handle grip.

In one embodiment, the actuator further comprises a fluid injection port wherein said fluid injection port is positioned at the distal end of said actuator and in-line with said handle body. In one embodiment, the needle includes a second lumen and said fluid injection port of said actuator is in fluid communication with said first lumen of said drive shaft which is in fluid communication with said second lumen of said needle.

In one embodiment, the injection needle device further comprises a sheath disposed over said drive shaft and having a proximal end and a distal end, wherein said proximal end of said sheath attaches to said distal end of said body and said spring coil is positioned within said distal end of said sheath. In various embodiments, an outer diameter of said sheath is 2.1 to 2.5 mm. In various embodiments, a length of said sheath is 220 to 260 mm.

In one embodiment, the injection needle device further comprises a collar positioned distal to said spring coil and having a proximal end and a distal end. In one embodiment, the spring coil and collar stabilize lateral movement of said needle and provide for longitudinal movement of said needle. In one embodiment, the distal end of said collar is dome shaped. In one embodiment, a distance said needle extends beyond the distal end of said collar is substantially equal to a distance said actuator is advanced into said handle. In one embodiment, the collar is attached to said spring coil. Optionally, in another embodiment, the injection needle device further comprises a sheath disposed over said drive shaft, said spring coil, and said collar, and the collar is not attached to said spring coil and is held in place by said sheath. In various embodiments, the needle has a minimum extendable length beyond the distal end of said collar of 2 to 4 mm. In various embodiments, the needle has a maximum extendable length beyond the distal end of said collar of 4 to 6 mm.

In one embodiment, each of said plurality of locking components has a length of 1 mm.

In various embodiments, the needle is any one of a 22 gauge or 25 gauge needle.

The present specification also discloses an injection needle device for use with an endoscope, said device comprising: a handle having a cylindrical body, an exterior surface, a proximal end, and a distal end, said handle further comprising a first locking member on said exterior surface and a compressible spring within said cylindrical body; an actuator having a proximal end and a distal end, wherein said actuator is coupled at said proximal end to the distal end of said cylindrical body of said handle, said actuator further comprising a second locking member having a plurality of locking components, each of said locking components being separated by an interval; a spring coil; a drive shaft having a first lumen, a proximal end, and a distal end, wherein said proximal end of said drive shaft attaches to said distal end of said actuator and wherein said distal end of said shaft extends within said spring coil; a sheath disposed over said drive shaft and having a proximal end and a distal end, wherein said proximal end of said sheath attaches to said distal end of said body and said spring coil is positioned within said distal end of said sheath; a flexible elongate tube disposed over a proximal portion of said sheath and having a proximal end and a distal end, said proximal end of said tube attached to said distal end of said handle, said tube further having a decreasing outer diameter as it extends from said proximal end to said distal end; and a needle attached to said distal end of said drive shaft, wherein said actuator is configured such that, upon advancement, said first locking member traverses said plurality of locking components of said second locking member and intervals therebetween, engaging one of the plurality of locking components of said second locking member to lock said actuator in place and said proximal end of said actuator telescopes into said body of said handle and compresses said compressible spring causing said needle to move from a first position to a second position, separated by a distance, wherein the distance is a predefined distance that is determinable based upon a number of said intervals traversed by the first locking member.

The present specification also discloses a method of using an injection needle device with an endoscope, said method comprising the steps of: providing an injection needle device comprising: a handle having a cylindrical body, an exterior surface, a proximal end, and a distal end, said handle further comprising a first locking member on said exterior surface and a compressible spring within said cylindrical body; an actuator having a proximal end and a distal end, wherein said actuator is coupled at said proximal end to the distal end of said cylindrical body of said handle, said actuator further comprising a second locking member having a plurality of locking components, each of said locking components being separated by an interval, said actuator further comprising a fluid injection port in fluid communication with a first lumen of a drive shaft; a spring coil; a drive shaft having a first lumen, a proximal end, and a distal end, wherein said proximal end of said drive shaft attaches to said distal end of said actuator and wherein said distal end of said shaft extends within said spring coil; and a needle attached to said distal end of said drive shaft and having a second lumen in communication with said first lumen of said actuator, wherein said actuator is configured such that, upon advancement, said first locking member traverses said plurality of locking components of said second locking member and intervals therebetween, engaging one of the plurality of locking components of said second locking member to lock said actuator in place and said proximal end of said actuator telescopes into said body of said handle and compresses said compressible spring causing said needle to move from a first position to a second position, separated by a distance, wherein the distance is a predefined distance that is determinable based upon a number of said intervals traversed by the first locking member; inserting said injection needle device into a working channel of an endoscope; positioning said distal end of said drive shaft proximate a target tissue; extending said needle by advancing said actuator such that one of said plurality of locking elements on said second locking member engages said first locking member, wherein the length of said extended needle corresponds to the position of said one of said plurality of locking elements; advancing the injection needle device to pierce said target tissue; injecting fluid into said injection port, through said actuator, drive shaft, and needle, and into said target tissue; pulling back the injection needle device to remove needle from said target tissue; and manipulating said first locking member to release said second locking member, allowing said compressible spring to expand and extend actuator proximally, retracting said needle.

In various embodiments, the method of using an injection needle device with an endoscope further comprises repeating the steps of positioning said distal end of said drive shaft proximate a target tissue through manipulating said first locking member to release said second locking member, wherein said needle is extended a plurality of lengths corresponding to the locking component position that is engaged with said first locking member.

The aforementioned and other embodiments of the present invention shall be described in greater depth in the drawings and detailed description provided below.

### BRIEF DESCRIPTION OF THE DRAWINGS

These and other features and advantages of the present invention will be further appreciated, as they become better understood by reference to the detailed description when considered in connection with the accompanying drawings:
Figure 1 is an illustration of one embodiment of the endoscopic injection needle device with the needle retracted into the sheath;
Figure 2 is a side view illustration of the same embodiment of the endoscopic injection needle device with the needle withdrawn into the sheath of Figure 1;
Figure 3 is a close-up illustration of the distal end of the sheath of the same embodiment of the endoscopic injection needle device of Figure 1, depicting the drive shaft, spring coil, and collar therewithin;
Figure 4 is a side view illustration of the same embodiment of the endoscopic injection needle device of Figure 1, depicting the needle extended to its minimum lockable distance from the distal end of the collar;
Figure 5 is a close-up illustration of the distal end of the sheath of the same embodiment of the endoscopic needle injection device of Figure 4, depicting the needle extended to its minimum lockable distance from the distal end of the collar;
Figure 6 is a side view illustration of the same embodiment of the endoscopic injection needle device of Figure 1, depicting the needle extended to a length between its minimum lockable distance and maximum lockable distance from the distal end of the collar;
Figure 7 is a close-up illustration of the distal end of the sheath of the same embodiment of the endoscopic needle injection device of Figure 6, depicting the needle extended to a length between its minimum lockable distance and maximum lockable distance from the distal end of the collar;
Figure 8 is a side view illustration of the same embodiment of the endoscopic injection needle device of Figure 1, depicting the needle extended to its maximum lockable distance from the distal end of the collar;
Figure 9 is a side view illustration of the same embodiment of the endoscopic injection needle device of Figure 1, depicting the needle extended to its maximum lockable distance from the distal end of the collar and the locking bar fully engaged with the locking port;
Figure 10 is a close-up illustration of the distal end of the sheath of the same embodiment of the endoscopic needle injection device of Figure 8, depicting the needle extended to its maximum lockable distance from the distal end of the collar; and,
Figure 11 is a flowchart illustrating one embodiment of the steps included during a surgical procedure using the endoscopic injection needle device of the present specification.

### DETAILED DESCRIPTION

The present specification discloses an endoscopic injection needle device including a spring and reinforced collar at its distal end used to stabilize and deploy the needle in a reliable and consistent manner. The present invention is directed toward multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. The general principles defined herein may be applied to other embodiments and applications without departing from the spirit and scope of the invention. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purpose of clarity, details relating to technical material that is known in the technical fields related to the invention have not been described in detail so as not to unnecessarily obscure the present invention.

The device includes a handle comprising a cylindrical body having an exterior surface, a proximal end, and a distal end. At its distal end, the handle further comprises a locking port on its exterior surface. In one embodiment, the handle also includes a plurality of ridges along opposite sides of its exterior surface that function as grips, providing enhanced ergonomics for greater comfort and control. An actuator having a proximal end and a distal end is coupled to the proximal end of the handle body and is movable longitudinally into a plurality of locked positions. The proximal end of the actuator includes a fluid injection port that is in-line with the handle body. The in-line injection port promotes direct and unimpeded fluid flow. The proximal end of the actuator also includes a mechanism to secure a syringe in-line with the injection port. In one embodiment, the securing mechanism is of a luer lock type. The distal end of the actuator extends inside the body of the handle. In one embodiment, the actuator is a plunger that is spring loaded by a spring within the handle body and telescopes into the body of the handle. The actuator further comprises a locking bar in line with the locking port of the handle. The locking bar includes a series of teeth that engage the locking port as the actuator is advanced distally into the handle body. In one embodiment, a flexible tube is attached to the distal end of the handle body and extends distally therefrom. The flexible tube is tapered, having a diameter that decreases as it extends distally and facilitates placement of the device in an endoscope.

An elongate flexible sheath having a sheath lumen within, a proximal end, and a distal end is attached to the distal end of the handle body and extends distally therefrom, passing through the flexible tube. An elongate, flexible drive shaft having a shaft lumen within a proximal end and a distal end is disposed within the sheath. In one embodiment, the drive shaft is composed of plastic. The outer surface of the drive shaft is in contact with the inner surface of the sheath and the drive shaft is movable longitudinally within the sheath. The proximal end of the drive shaft extends beyond the proximal end of the sheath into the body of the handle and is attached to the distal end of the actuator therewithin. The distal end of the shaft has a needle attached to it and is positioned within the sheath at a point proximate the distal end of the sheath. In another embodiment, the drive shaft is replaced by a flexible metal wire. A spring coil having a lumen, a proximal end, and a distal end is also positioned within the distal end of the sheath and has the distal end of the shaft with attached needle extending through it. In one embodiment, the spring coil is a reinforced stainless steel spring coil. A collar having a proximal end and a distal end is attached at its proximal end to the distal end of the spring coil. The proximal end of the collar is positioned within the sheath and the distal end of the collar extends beyond the distal end of the sheath and is rounded to have a dome shape. The collar houses the needle and includes an opening at its distal end for passage of said needle. In one embodiment, the collar is a reinforced stainless steel collar. The spring coil and collar provide greater sheath stability for consistent needle deployment, including in tortuous anatomy. The collar also prevents the needle from being inadvertently advanced through the sheath wall. The spring coil also serves to enhance user comfort and efficiency by facilitating needle deployment and retraction.

To extend the needle, a user advances the actuator into the body of the handle. As the actuator is moved into the handle body, it advances the drive shaft within the sheath, causing extension of the needle beyond the dome of the collar. Using a drive shaft within a sheath to extend the needle reduces the coefficient of friction to ensure repeatedly smooth needle deployment and retraction. The needle begins to extend beyond the dome as the locking bar of the actuator approaches the locking port of the handle. The teeth of the locking bar engage the locking port and lock the actuator in place. The lock secures needle penetration and prevents needle bounce back. In one embodiment, once the needle begins to extend, its exposed length corresponds to the additional distance that the actuator is advanced. Each of the teeth on the locking bar is capable of engaging the locking port. Therefore, in one embodiment, as the actuator is advanced a distance equal to the length of one tooth on the locking bar, the needle is extended the same distance beyond the collar. In one embodiment, that distance is 1 mm. In various embodiments, the needle has a minimum extension length between 2 and 4 mm beyond the distal end of the collar. In one embodiment, the needle has a minimum extension length of 3 mm beyond the distal end of the collar. In various embodiments, the needle has a maximum extension length between 4 and 6 mm beyond the distal end of the collar. In one embodiment, the needle has a maximum extension length of 5 mm beyond the distal end of the collar. In one embodiment, the needle is a 22 gauge needle. In another embodiment, the needle is a 25 gauge needle. To retract the needle, a user presses down on the locking port. This action releases the teeth of the locking bar and the spring within the handle body pushes the actuator proximally, telescoping it back to its original position. As the actuator returns to its original position, it pulls on the drive shaft, causing the attached needle to retract back into the sheath.

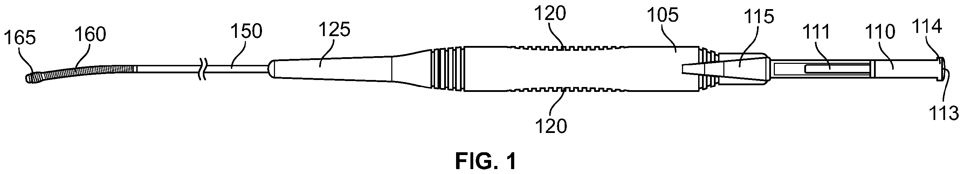

Figure 1 is an illustration of one embodiment of the endoscopic injection needle device with the needle retracted into the sheath 150. The device includes a handle 105 having a cylindrical body with a proximal end and a distal end. The handle 105 includes a locking port 115 at its proximal end and a flexible tapered tube 125 at its distal end. The handle 105 also includes ridges 120 extending along both sides which function as a grip. Coupled to the proximal end of the handle 105 is an actuator 110 having a proximal end and a distal end. The distal end of the actuator 110 extends into the body of the handle 105. The proximal end of the actuator 110 includes an injection port 113 in-line with the handle 105 body and a luer lock connection 114 for the attachment of a syringe. Also depicted is the locking bar 111 of the actuator 110.

A flexible sheath 150 is attached to the distal end of the handle 105 body and passes through the flexible tapered tube 125. In various embodiments, the sheath has an outside diameter ranging between 2.1 and 2.5 mm. In one embodiment, the sheath has an outside diameter of 2.3 mm. In various embodiments, the sheath has a length between 220 and 260 mm. In one embodiment, the sheath has a length of 240 mm.

A spring coil 160 is positioned within the distal end of the sheath 150. A collar 165 is also positioned within the distal end of the sheath 150, just distal to the spring coil 160, and has a dome shaped distal end that extends beyond the distal end of the sheath 150. A drive shaft (not visible in Figure 1) having a proximal end and a distal end, is attached at its proximal end to the distal end of the actuator 110, extends through the handle 105 body, sheath 150, and spring coil 160, and has a needle attached to its distal end. When withdrawn into the sheath 150, the needle is housed within the collar 165.

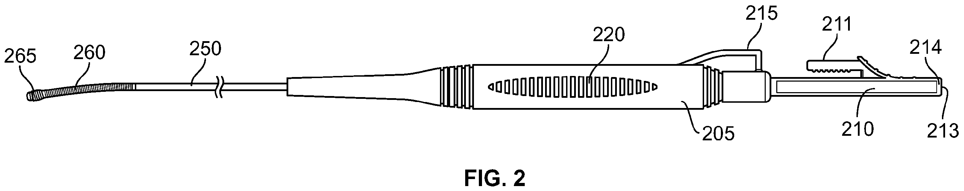

Figure 2 is a side view illustration of the same embodiment of the endoscopic injection needle device with the needle withdrawn into the sheath 250 of Figure 1. Referring to Figure 2, the teeth of the locking bar 211 are depicted on the actuator 210. The ridges 220 on one side of the handle 205 are also better visualized in Figure 2. The locking port 215 of the handle 205 is depicted elevated from the surface of the handle 205 body. Also depicted are the sheath 250, spring coil 260, and collar 265.

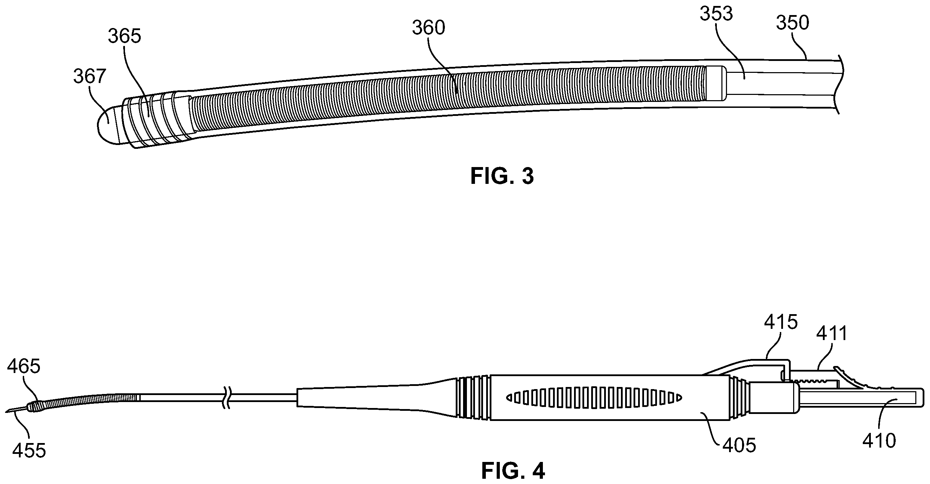

Figure 3 is a close-up illustration of the distal end of the sheath 350 of the same embodiment of the endoscopic injection needle device of Figure 1, depicting the drive shaft 353, spring coil 360, and collar 365 therewithin. Referring to Figure 3, the needle (not shown) is withdrawn into the sheath. The drive shaft 353 extends through the spring coil 360 and has a needle attached to its distal end. The collar 365 houses the needle and includes a dome shaped distal end 367 that extends beyond the distal end of the sheath. In one embodiment, the collar 365 is positioned just distal to the spring coil 360 and the two are not attached. In another embodiment, the proximal end of the collar 365 is attached to the distal end of the spring coil 360.

Figure 4 is a side view illustration of the same embodiment of the endoscopic injection needle device of Figure 1, depicting the needle 455 extended to its minimum lockable distance from the distal end of the collar 465. The actuator 410 has been advanced such that the first tooth of its locking bar 411 has engaged a slot in the locking port 415 of the handle 405, thereby locking the actuator 410 in place and extending the needle 455 to its minimum lockable distance.

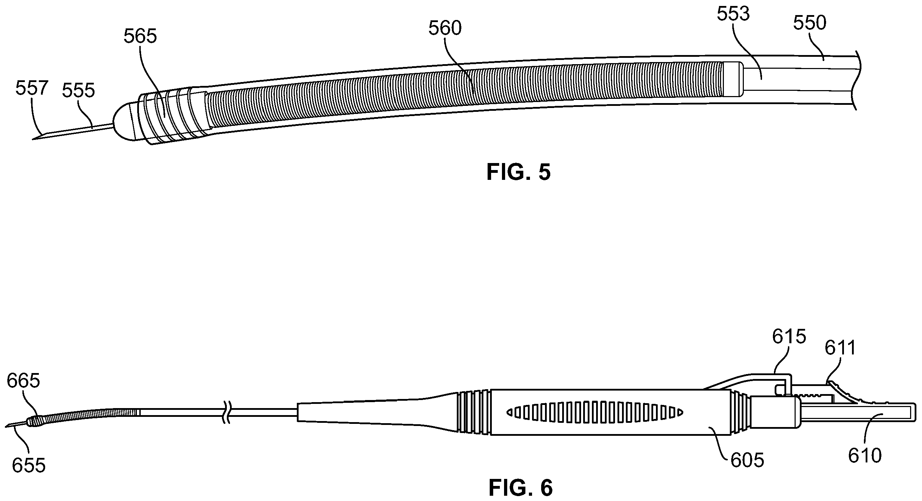

Figure 5 is a close-up illustration of the distal end of the sheath 550 of the same embodiment of the endoscopic needle injection device of Figure 4, depicting the needle 555 extended to its minimum lockable distance from the distal end of the collar 565. Also depicted are the drive shaft 553 and spring coil 560 extending within the sheath 550. The needle 555 includes a beveled tip 557 for piercing target tissues. The needle also includes a lumen therewithin for the injection of fluids into the target tissues. In one embodiment, the needle is a 22 gauge needle. In another embodiment, the needle is a 25 gauge needle. In various embodiments, the minimum lockable exposed length of the needle ranges from 2 to 4 mm. In one embodiment, the minimum lockable exposed length of the needle is 3 mm.

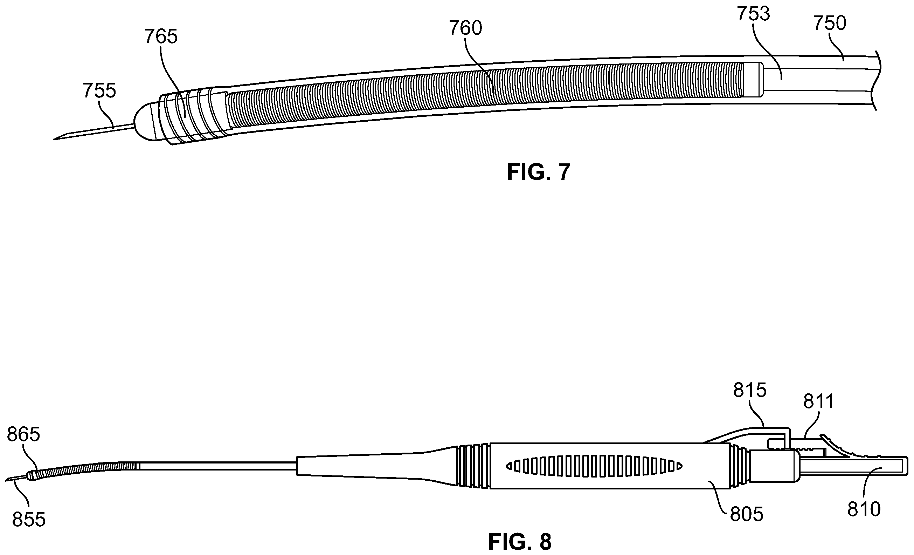

Figure 6 is a side view illustration of the same embodiment of the endoscopic injection needle device of Figure 1, depicting the needle 655 extended to a length between its minimum lockable distance and maximum lockable distance from the distal end of the collar 665. The actuator 610 has been advanced such that the second tooth of its locking bar 611 has engaged a slot in the locking port 615 of the handle 605, thereby locking the actuator 610 in place and extending the needle 655 to its said distance. Figure 7 is a close-up illustration of the distal end of the sheath 750 of the same embodiment of the endoscopic needle injection device of Figure 6, depicting the needle 755 extended to a length between its minimum lockable distance and maximum lockable distance from the distal end of the collar 765. Also depicted are the drive shaft 753 and spring coil 760 extending within the sheath 750.

Figure 8 is a side view illustration of the same embodiment of the endoscopic injection needle device of Figure 1, depicting the needle 855 extended to its maximum lockable distance from the distal end of the collar 865. The actuator 810 has been advanced such that the third tooth of its locking bar 811 has engaged a slot in the locking port 815 of the handle 805, thereby locking the actuator 810 in place and extending the needle 855 to its maximum lockable distance.

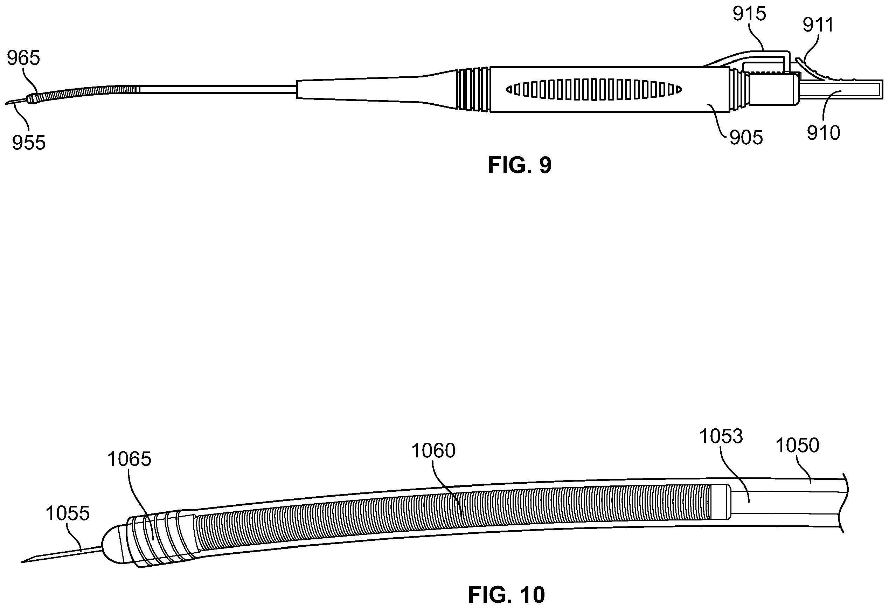

Figure 9 is a side view illustration of the same embodiment of the endoscopic injection needle device of Figure 1, depicting the needle 955 extended to its maximum lockable distance from the distal end of the collar 965 and the locking bar 911 fully engaged with the locking port 915. The actuator 910 has been advanced such that the last tooth of its locking bar 911 has engaged a slot in the locking port 915 of the handle 905. This does not advance the needle any farther than the configuration shown in Figure 8. Therefore, in the pictured embodiment, advancing the locking bar beyond the third tooth does not have an effect on the exposed length of the needle. However, other embodiments are envisioned wherein each advancement of the locking bar by one tooth further extends the needle by the same distance for every tooth of the locking bar.

Figure 10 is a close-up illustration of the distal end of the sheath 1050 of the same embodiment of the endoscopic needle injection device of Figure 8, depicting the needle 1055 extended to its maximum lockable distance from the distal end of the collar 1065. Also depicted are the drive shaft 1053 and spring coil 1060 extending within the sheath 1050. In various embodiments, the maximum lockable exposed length of the needle ranges from 4 to 6 mm. In one embodiment, the maximum lockable exposed length of the needle is 5 mm.

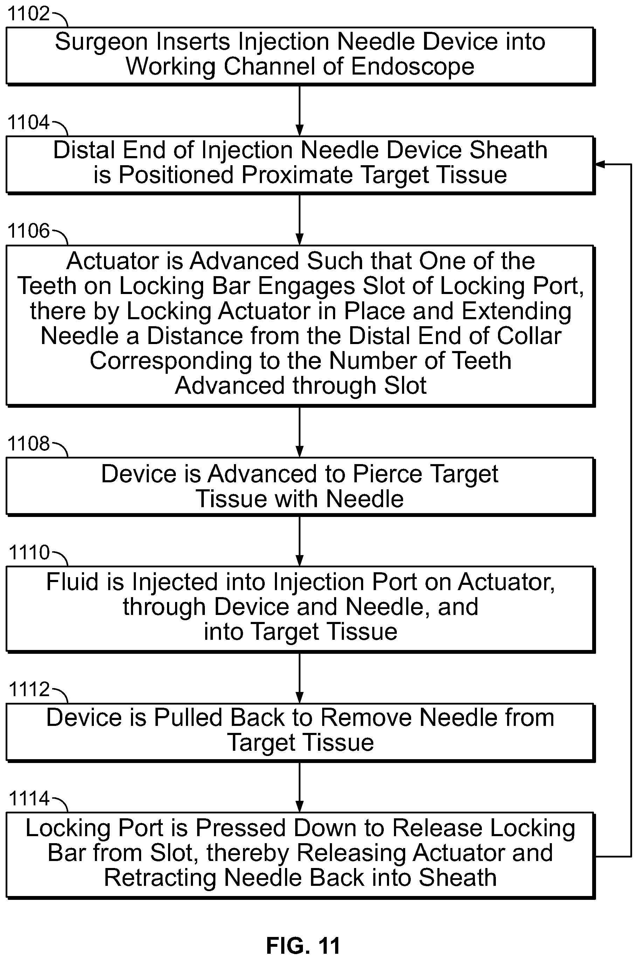

Figure 11 is a flowchart illustrating one embodiment of the steps included during a surgical procedure using the endoscopic injection needle device of the present specification. At step 1102, a surgeon inserts the injection needle device into a working channel of an endoscope. The distal end of the device sheath is then positioned proximate the target tissue at step 1104. At step 1106, the actuator is advanced such that one of the teeth on the locking bar engages the slot of the locking port, thereby locking the actuator in place and extending the needle a distance from the distal end of the collar that corresponds to the number of teeth advanced through the slot. Then, at step 1108, the device is advanced to pierce the target tissue with the needle. At step 1110, using a syringe, fluid is injected into the in-line injection port on the actuator, through the device and needle, and into the target tissue. The device is then pulled back at step 1112 to remove the needle from the target tissue. At step 1114, the locking port is pressed down to release the locking bar from the slot, thereby releasing the actuator and retracting the needle back into the sheath. Steps 1104 through 1114 can be repeated multiple times in the procedure with the assurance that the needle will deploy and retract correctly each time due to the increase stability imparted by the spring coil and collar.

The above examples are merely illustrative of the many applications of the system of the present invention. Although only a few embodiments of the present invention have been described herein, it should be understood that the present invention might be embodied in many other specific forms without departing from scope of the invention. Therefore, the present examples and embodiments are to be considered as illustrative and not restrictive, and the invention may be modified within the scope of the appended claims.

## Claims

1. An injection needle device for use with an endoscope, said device comprising:
a handle (105, 205, 405, 605, 805, 905) having a cylindrical body, an exterior surface, a proximal end, and a distal end;
a first locking member (115, 215, 415, 615, 815, 915) on said exterior surface of said handle;
a compressible spring within said cylindrical body of said handle;
an actuator (110, 210, 410, 610, 810, 910) having a proximal end and a distal end, wherein said actuator is coupled at its proximal end to the distal end of said cylindrical body of said handle;
a sheath (150, 250, 350, 550, 750, 1050) having a proximal end and a distal end, wherein said proximal end of said sheath is attached to said distal end of said cylindrical body of said handle;
a spring coil (160, 260, 360, 560, 760, 1060) positioned within said distal end of said sheath;
a drive shaft (353, 553, 753, 1053) extending through said cylindrical body of said handle and said sheath and having a first lumen, a proximal end, and a distal end, wherein said proximal end of said drive shaft attaches to said distal end of said actuator and wherein said distal end of said shaft extends within said spring coil;
a needle (455, 555, 655, 755, 855, 955, 1055) attached to said distal end of said drive shaft;
**characterized in that**
a second locking member (111, 211, 411, 611, 811, 911) is positioned on said actuator (110, 210, 410, 610, 810, 910) and having a plurality of locking components, each of said locking components being separated by an interval; and
a collar (165, 265, 365, 465, 565, 665, 765, 865, 965, 1065) is positioned within said distal end of said sheath (150, 250, 350, 550, 750, 1050) distal to said spring coil (160, 260, 360, 560, 760, 1060) and having a proximal end and a distal end,
wherein, when said actuator is advanced into said cylindrical body of said handle (105, 205, 405, 605, 805, 905), said first locking member (115, 215, 415, 615, 815, 915) traverses said plurality of locking components of said second locking member and intervals therebetween, engaging one of the plurality of locking components of said second locking member to lock said actuator in place and said proximal end of said actuator telescopes into said body of said handle and compresses said compressible spring causing said drive shaft (353, 553, 753, 1053) to move within said sheath and said needle (455, 555, 655, 755, 855, 955, 1055) to move from a first position to a second position, separated by a distance, wherein the distance is a predefined distance that is determinable based upon a number of said intervals traversed by the first locking member, further wherein said spring coil and said collar stabilize lateral movement of said needle and provide for longitudinal movement of said needle.

2. The injection needle device of claim 1, further comprising a plurality of ridges (120, 220) on at least one side of said exterior surface of said handle (105, 205, 405, 605, 805, 905) to form a handle grip.

3. The injection needle device of claim 1, wherein said actuator (110, 210, 410, 610, 810, 910) further comprises a fluid injection port (113) and wherein said fluid injection port is positioned at the distal end of said actuator and in-line with said handle (105, 205, 405, 605, 805, 905) body.

4. The injection needle device of claim 3, wherein said needle (455, 555, 655, 755, 855, 955, 1055) includes a second lumen and said fluid injection port (113) of said actuator (110, 210, 410, 610, 810, 910) is in fluid communication with said first lumen of said drive shaft (353, 553, 753, 1053) which is in fluid communication with said second lumen of said needle.

5. The injection needle device of claim 1, wherein an outer diameter of said sheath (150, 250, 350, 550, 750, 1050) is 2.1 to 2.5 mm.

6. The injection needle device of claim 1, wherein a length of said sheath (150, 250, 350, 550, 750, 1050) is 220 to 260 mm.

7. The injection needle device of claim 1, wherein said distal end of said collar (165, 265, 365, 465, 565, 665, 765, 865, 965, 1065) is dome shaped.

8. The injection needle device of claim 1, wherein a distance said needle (455, 555, 655, 755, 855, 955, 1055) extends beyond the distal end of said collar (165, 265, 365, 465, 565, 665, 765, 865, 965, 1065) is substantially equal to a distance said actuator (110, 210, 410, 610, 810, 910) is advanced into said handle (105, 205, 405, 605, 805, 905).

9. The injection needle device of claim 1, wherein said collar (165, 265, 365, 465, 565, 665, 765, 865, 965, 1065) is attached to said spring coil (160, 260, 360, 560, 760, 1060).

10. The injection needle device of claim 1, wherein said needle (455, 555, 655, 755, 855, 955, 1055) has a minimum extendable length beyond the distal end of said collar (165, 265, 365, 465, 565, 665, 765, 865, 965, 1065) of 2 to 4 mm.

11. The injection needle device of claim 1, wherein said needle (455, 555, 655, 755, 855, 955, 1055) has a maximum extendable length beyond the distal end of said collar (165, 265, 365, 465, 565, 665, 765, 865, 965, 1065) of 4 to 6 mm.

12. The injection needle device of claim 1, wherein each of said plurality of locking components has a length of 1 mm.

13. The injection needle device of claim 1, wherein said needle (455, 555, 655, 755, 855, 955, 1055) is any one of a 22 gauge or 25 gauge needle.

14. The injection needle device of claim 1, said device further comprising:
a flexible elongate tube disposed over a proximal portion of said sheath (150, 250, 350, 550, 750, 1050) and having a proximal end and a distal end, said proximal end of said tube attached to said distal end of said handle (105, 205, 405, 605, 805, 905), said tube further having a decreasing outer diameter as it extends from said proximal end to said distal end.

## Patentansprüche

1. Injektionsnadelvorrichtung zur Verwendung mit einem Endoskop, wobei die Vorrichtung aufweist:
einen Griff (105, 205, 405, 605, 805, 905), der einen zylindrischen Körper, eine Außenfläche, ein proximales Ende und ein distales Ende aufweist;
ein erstes Verriegelungselement (115, 215, 415, 615, 815, 915) an der Außenfläche des Griffs;
eine zusammendrückbare Feder innerhalb des zylindrischen Körpers des Griffs;
ein Betätigungselement (110, 210, 410, 610, 810, 910), das ein proximales Ende und ein distales Ende aufweist, wobei das Betätigungselement an seinem proximalen Ende mit dem distalen Ende des zylindrischen Körpers des Griffs gekoppelt ist;
eine Hülle (150, 250, 350, 550, 750, 1050), die ein proximales Ende und ein distales Ende aufweist, wobei das proximale Ende der Hülle am distalen Ende des zylindrischen Körpers des Griffs angebracht ist;
eine Schraubenfeder (160, 260, 360, 560, 760, 1060), die innerhalb des distalen Endes der Hülle angeordnet ist;
einen Antriebsschaft (353, 553, 753, 1053), der sich durch den zylindrischen Körper des Griffs und die Hülle erstreckt und ein erstes Lumen, ein proximales Ende und ein distales Ende aufweist, wobei das proximale Ende des Antriebsschafts am distalen Ende des Betätigungselements angebracht ist und wobei sich das distale Ende des Schafts in die der Schraubenfeder erstreckt;
eine Nadel (455, 555, 655, 755, 855, 955, 1055), die an das distale Ende des Antriebsschafts angebracht ist;
**dadurch gekennzeichnet, dass**
ein zweites Verriegelungselement (111, 211, 411, 611, 811, 911) am Betätigungselement (110, 210, 410, 610, 810, 910) angeordnet ist und mehrere Verriegelungskomponenten aufweist, wobei jede der Verriegelungskomponenten durch einen Zwischenraum getrennt ist; und
ein Bund (165, 265, 365, 465, 565, 665, 765, 865, 965, 1065) im distalen Ende der Hülle (150, 250, 350, 550, 750, 1050) distal zur Schraubenfeder (160, 260, 360, 560, 760, 1060) angeordnet ist und ein proximales Ende und ein distales Ende aufweist,
wobei wenn das Betätigungselement in den zylindrischen Körper des Griffs (105, 205, 405, 605, 805, 905) vorgeschoben wird, das erste Verriegelungselement (115, 215, 415, 615, 815, 915) die mehreren Verriegelungskomponenten des zweiten Verriegelungselements und die Zwischenräume dazwischen durchquert, wobei es mit einer der mehreren Verriegelungskomponenten des zweiten Verriegelungselements in Eingriff tritt, um das Betätigungselement an Ort und Stelle zu verriegeln, und das proximale Ende des Betätigungselements in den Körper des Griffs teleskopiert und die zusammendrückbare Feder zusammendrückt, wodurch der Antriebsschaft (353, 553, 753, 1053) veranlasst wird, sich in die Hülle zu bewegen und die Nadel (455, 555, 655, 755, 855, 955, 1055) veranlasst wird, sich von einer ersten Position zu einer zweiten Position zu bewegen, die durch einen Abstand getrennt sind, wobei der Abstand ein vordefinierter Abstand ist, der beruhend auf der Anzahl der Zwischenräume bestimmbar ist, die durch das erste Verriegelungselement durchquert werden, wobei ferner die Schraubenfeder und der Bund eine laterale Bewegung der Nadel stabilisieren und für eine longitudinale Bewegung der Nadel sorgen.

2. Injektionsnadelvorrichtung nach Anspruch 1, die ferner mehrere Rippen (120, 220) auf mindestens einer Seite der Außenfläche des Griffs (105, 205, 405, 605, 805, 905) aufweist, um einen Handgriff zu bilden.

3. Injektionsnadelvorrichtung nach Anspruch 1, wobei das Betätigungselement (110, 210, 410, 610, 810, 910) ferner einen Fluidinjektionsanschluss (113) aufweist und wobei der Fluidinjektionsanschluss am distalen Ende des Betätigungselements und in einer Linie mit dem Griffkörper (105, 205, 405, 605, 805, 905) angeordnet ist.

4. Injektionsnadelvorrichtung nach Anspruch 3, wobei die Nadel (455, 555, 655, 755, 855, 955, 1055) ein zweites Lumen aufweist und der Fluidinjektionsanschluss (113) des Betätigungselements (110, 210, 410, 610, 810, 910) in Fluidverbindung mit dem ersten Lumen des Antriebsschafts (353, 553, 753, 1053) steht, das mit dem zweiten Lumen der Nadel in Fluidverbindung steht.

5. Injektionsnadelvorrichtung nach Anspruch 1, wobei ein Außendurchmesser der Hülle (150, 250, 350, 550, 750, 1050) 2,1 bis 2,5 mm beträgt.

6. Injektionsnadelvorrichtung nach Anspruch 1, wobei eine Länge der Hülle (150, 250, 350, 550, 750, 1050) 220 bis 260 mm beträgt.

7. Injektionsnadelvorrichtung nach Anspruch 1, wobei das distale Ende des Bunds (165, 265, 365, 465, 565, 665, 765, 865, 965, 1065) kuppelförmig ist.

8. Injektionsnadelvorrichtung nach Anspruch 1, wobei eine Strecke, um die sich die Nadel (455, 555, 655, 755, 855, 955, 1055) über das distale Ende des Bunds (165, 265, 365, 465, 565, 665, 765, 865, 965, 1065) hinaus erstreckt, im Wesentlichen gleich einer Strecke ist, um die das Betätigungselement (110, 210, 410, 610, 810, 910) in den Griff (105, 205, 405, 605, 805, 905) vorgeschoben wird.

9. Injektionsnadelvorrichtung nach Anspruch 1, wobei der Bund (165, 265, 365, 465, 565, 665, 765, 865, 965, 1065) an der Schraubenfeder (160, 260, 360, 560, 760, 1060) angebracht ist.

10. Injektionsnadelvorrichtung nach Anspruch 1, wobei die Nadel (455, 555, 655, 755, 855, 955, 1055) eine minimale ausfahrbare Länge über das distale Ende des Bunds (165, 265, 365, 465, 565, 665, 765, 865, 965, 1065) hinaus von 2 bis 4 mm aufweist.

11. Injektionsnadelvorrichtung nach Anspruch 1, wobei die Nadel (455, 555, 655, 755, 855, 955, 1055) eine maximale ausfahrbare Länge über das distale Ende des Bunds (165, 265, 365, 465, 565, 665, 765, 865, 965, 1065) hinaus von 4 bis 6 mm aufweist.

12. Injektionsnadelvorrichtung nach Anspruch 1, wobei jede der mehreren Verriegelungskomponenten eine Länge von 1 mm aufweist.

13. Injektionsnadelvorrichtung nach Anspruch 1, wobei die Nadel (455, 555, 655, 755, 855, 955, 1055) irgendeine einer 22-Gauge- oder 25-Gauge-Nadel ist.

14. Injektionsnadelvorrichtung nach Anspruch 1, wobei die Vorrichtung ferner aufweist:
eine flexible längliche Röhre, die über einem proximalen Abschnitt der Hülle (150, 250, 350, 550, 750, 1050) angeordnet ist und ein proximales Ende und ein distales Ende aufweist, wobei das proximale Ende der Röhre am distalen Ende des Griffs (105, 205, 405, 605, 805, 905) angebracht ist, wobei die Röhre ferner einen abnehmenden Außendurchmesser, vom proximalen Ende zum distalen Ende aufweist.

## Revendications

1. Dispositif d'aiguille pour injection destiné à être utilisé avec un endoscope, ledit dispositif comprenant :
une poignée (105, 205, 405, 605, 805, 905) ayant un corps cylindrique, une surface extérieure, une extrémité proximale et une extrémité distale ;
un premier élément de verrouillage (115, 215, 415, 615, 815, 915) sur la surface extérieure de la poignée ;
un ressort compressible à l'intérieur du corps de poignée cylindrique ;
un actionneur (110, 210, 410, 610, 810, 910) ayant une extrémité proximale et une extrémité distale, ledit actionneur étant relié par son extrémité proximale à l'extrémité distale du corps de poignée cylindrique ;
une gaine (150, 250, 350, 550, 750, 1050) ayant une extrémité proximale et une extrémité distale, l'extrémité proximale de la gaine étant raccordée à l'extrémité distale du corps de poignée cylindrique ;
un ressort hélicoïdal (160, 260, 360, 560, 760, 1060) disposé à l'intérieur de l'extrémité distale de la gaine ;
une tige d'entraînement (353, 553, 753, 1053) s'étendant dans le corps de poignée cylindrique et la gaine et ayant une première lumière, une extrémité proximale et une extrémité distale, l'extrémité proximale de la tige d'entraînement étant raccordée à l'extrémité distale de l'actionneur et l'extrémité distale de la tige s'étendant à l'intérieur du ressort hélicoïdal ;
une aiguille (455, 555, 655, 755, 855, 955, 1055) raccordée à l'extrémité distale de la tige d'entraînement ;
**caractérisé**
**en ce qu'**un deuxième élément de verrouillage (111, 211, 411, 611, 811, 911) est disposé sur l'actionneur (110, 210, 410, 610, 810, 910) et présente une pluralité de composants de verrouillage, lesdits composants de verrouillage étant séparés les uns des autres par un intervalle ; et
**en ce qu'**un collier (165, 265, 365, 465, 565, 665, 765, 865, 965, 1065) est disposé à l'intérieur de l'extrémité distale de la gaine (150, 250, 350, 550, 750, 1050), distalement par rapport au ressort hélicoïdal (160, 260, 360, 560, 760, 1060) et a une extrémité proximale et une extrémité distale,
et, si l'actionneur est avancé dans le corps de poignée cylindrique (105, 205, 405, 605, 805, 905), le premier élément de verrouillage (115, 215, 415, 615, 815, 915) traverse la pluralité de composants de verrouillage du deuxième élément de verrouillage et les intervalles entre ceux-ci, mettant en prise un composant de la pluralité de composants de verrouillage du deuxième élément de verrouillage afin de verrouiller l'actionneur sur place, l'extrémité proximale de l'actionneur télescope dans le corps de la poignée et comprime le ressort compressible, la tige d'entraînement (353, 553, 753, 1053) étant ainsi entraînée à se déplacer à l'intérieur de la gaine, et l'aiguille (455, 555, 655, 755, 855, 955, 1055) à se déplacer d'une première position vers une deuxième position séparées par une distance, ladite distance étant une distance prédéfinie déterminable sur la base du nombre d'intervalles traversés par le premier élément de verrouillage, le ressort hélicoïdal et le collier stabilisant en outre le mouvement latéral de l'aiguille et assurant le déplacement longitudinal de l'aiguille.

2. Dispositif d'aiguille pour injection selon la revendication 1, comprenant en outre une pluralité de nervures (120, 220) sur au moins un côté de la surface extérieure de la poignée (105, 205, 405, 605, 805, 905) pour former une structure de préhension de la poignée.

3. Dispositif d'aiguille pour injection selon la revendication 1, où l'actionneur (110, 210, 410, 610, 810, 910) comprend en outre un orifice d'injection de fluide (113) et où ledit orifice d'injection de fluide est présenté à l'extrémité distale de l'actionneur, en ligne avec le corps de poignée (105, 205, 405, 605, 805, 905).

4. Dispositif d'aiguille pour injection selon la revendication 3, où l'aiguille (455, 555, 655, 755, 855, 955, 1055) comprend une deuxième lumière et l'orifice d'injection de fluide (113) de l'actionneur (110, 210, 410, 610, 810, 910) est en communication fluidique avec la première lumière de la tige d'entraînement (353, 553, 753, 1053), laquelle est en communication fluidique avec la deuxième lumière de l'aiguille.

5. Dispositif d'aiguille pour injection selon la revendication 1, où le diamètre extérieur de la gaine (150, 250, 350, 550, 750, 1050) est compris entre 2,1 et 2,5 mm.

6. Dispositif d'aiguille pour injection selon la revendication 1, où la longueur de la gaine (150, 250, 350, 550, 750, 1050) est comprise entre 220 et 260 mm.

7. Dispositif d'aiguille pour injection selon la revendication 1, où l'extrémité distale du collier (165, 265, 365, 465, 565, 665, 765, 865, 965, 1065) est en forme de dôme.

8. Dispositif d'aiguille pour injection selon la revendication 1, où la longueur d'aiguille (455, 555, 655, 755, 855, 955, 1055) s'étendant au-delà de l'extrémité distale du collier (165, 265, 365, 465, 565, 665, 765, 865, 965, 1065) est sensiblement égale à la longueur d'actionneur (110, 210, 410, 610, 810, 910) étant avancé dans la poignée (105, 205, 405, 605, 805, 905).

9. Dispositif d'aiguille pour injection selon la revendication 1, où le collier (165, 265, 365, 465, 565, 665, 765, 865, 965, 1065) est fixé au ressort hélicoïdal (160, 260, 360, 560, 760, 1060).

10. Dispositif d'aiguille pour injection selon la revendication 1, où l'aiguille (455, 555, 655, 755, 855, 955, 1055) a une longueur d'extension minimale au-delà de l'extrémité distale du collier (165, 265, 365, 465, 565, 665, 765, 865, 965, 1065) comprise entre 2 et 4 mm.

11. Dispositif d'aiguille pour injection selon la revendication 1, où l'aiguille (455, 555, 655, 755, 855, 955, 1055) a une longueur d'extension maximale au-delà de l'extrémité distale du collier (165, 265, 365, 465, 565, 665, 765, 865, 965, 1065) comprise entre 4 et 6 mm.

12. Dispositif d'aiguille pour injection selon la revendication 1, où chaque composant de la pluralité de composants de verrouillage a une longueur de 1 mm.

13. Dispositif d'aiguille pour injection selon la revendication 1, où l'aiguille (455, 555, 655, 755, 855, 955, 1055) est une aiguille de calibre 22 ou une aiguille de calibre 25.

14. Dispositif d'aiguille pour injection selon la revendication 1, ledit dispositif comprenant en outre :
un tube allongé flexible présenté sur une partie proximale de la gaine (150, 250, 350, 550, 750, 1050) et ayant une extrémité proximale et une extrémité distale, l'extrémité proximale du tube étant raccordée à l'extrémité distale de la poignée (105, 205, 405, 605, 805, 905), le tube ayant en outre un diamètre extérieur décroissant entre son extrémité proximale et son extrémité distale.
